# EUROPÄISCHE PATENTSCHRIFT

(11) **EP 0 859 588 B1**
(45) Veröffentlichungstag und Bekanntmachung des Hinweises auf die Patenterteilung: **07.08.2002**
(21) Anmeldenummer: 96932532.3
(22) Anmeldetag: 14.09.1996
(51) Int. Cl.: A61K 7/13

(54) **OXIDATIONSFÄRBEMITTEL**
OXIDATION DYES
COLORANTS D'OXYDATION

(30) Priorität: 22.09.1995 DE 19535340
(43) Veröffentlichungstag der Anmeldung: 26.08.1998
(73) Patentinhaber: Henkel Kommanditgesellschaft auf Aktien, 40589 Düsseldorf-Holthausen (DE)
(72) Erfinder: HÖFFKES, Horst, D-40595 Düsseldorf (DE); HOLLENBERG, Detlef, D-40699 Erkrath (DE); MÜLLER, Reinhard, D-40723 Hilden (DE); ROSE, David, D-40723 Hilden (DE)
(86) Internationale Anmeldenummer: EP9604043
(87) Internationale Veröffentlichungsnummer: WO9710799

(56) Entgegenhaltungen:
- EP-A- 0 657 159
- EP-A- 0 663 204
- DE-A- 4 400 757
- DE-C- 4 301 663
- FR-A- 2 282 858

## Beschreibung

Die Erfindung betrifft Oxidationsfärbemittel zum Färben von Keratinfasern, die spezielle Entwickler-Kombinationen enthalten.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5 und 4-Amino-3-methylphenol, 2-Hydroxy-4,5,6-triaminopyrimidin, 2,4-Dihydroxy-5,6-diaminopyrimidin und 2,5,6-Triaminohydroxypyrimidin.

Als Kupplerkomponenten werden in der Regel m-Phenylendiaminderivate, Naphthole, Resorcin und Resorcinderivate, Pyrazolone, m-Aminophenole sowie Pyridin-Derivate verwendet. Als Kupplersubstanzen eignen sich insbesondere α-Naphthol, Pyrogallol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 2-Chlor-6-methyl-3-aminophenol, 2-Methylresorcin, 2,6-Diyhdroxypyridin und 2,6-Diaminopyridin.

Bezüglich weiterer Farbstoffkomponenten wird ausdrücklich auf die Colipa-Liste, herausgegeben vom Industrieverband Körperpflege und Waschmittel, Frankfurt, Bezug genommen.

Haarfärbemittel, welche Kombinationen aus Oxidationsfarbstoff vor produkten enthalten, sind beispielsweise aus den Offenlegungsschriffen DE-A-4 400 757, EP-A-0 657 159 und DE-A-3 917 304 bekannt.

Eine bestimmte Entwicklerkomponente kann durch Kombination mit verschiedenen Kupplern auch sehr unterschiedliche Farbnuancen bilden. Trotzdem gelingt es oft nicht, mit Hilfe einer einzigen Entwicklerkomponente zu der Vielzahl natürlicher Farbnuancen zu kommen. In der Praxis ist daher meist eine Kombination verschiedener Entwicklerkomponenten und Kupplerkomponenten erforderlich, um eine einzige, natürlich wirkende Färbung zu erhalten. Es besteht daher ständig Bedarf an neuen, verbesserten Kuppler/Entwickler-Kombinationen. Dies trifft insbesondere auch auf den Rot-Bereich zu, wo die gängigen Farbstoffe häufig noch nicht ganz befriedigende Reibechtheiten, Egalisiervermögen und Kaltwell- und Waschechtheiten aufweisen.

Es war daher die Aufgabe der vorliegenden Erfindung, neue Entwickler-Kombinationen und dazu passende Kupplerkomponenten zu finden, die die an Oxidationsfarbstoffvorprodukte zu stellenden Anforderungen in besonderem Maße erfüllen.

Überraschenderweise wurde nun gefunden, daß spezielle Kombinationen aus bekannten Entwicklerkomponenten, insbesondere in Kombination mit bestimmten, ebenfalls bekannten Kupplerkomponenten, zu intensiven Färbungen vor allem im Rot- und Orangebereich führen, die sich u.a. durch besonders gute Licht-, Wasch- und Reibechtheiten sowie ein überraschend hohes Egalisiervermögen auszeichnen.

Gegenstand der vorliegenden Erfindung sind daher Oxidationsfärbemittel zum Färben von Keratinfasern enthaltend Kupplerkomponenten und Entwicklerkomponenten in einem wasserhaltigen Träger, die als Entwicklerkomponenten mindestens ein Pyrimidin-Derivat mit 2 - 4 Aminosubstituenten und 0 - 2 Hydroxysubstituenten am Pyrimidin-Ring in Kombination mit 1-(2'-Hydroxyethyl)-2,5-diaminobenzol enthalten.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

In den erfindungsgemäßen Oxidationsfärbemitteln werden als Entwicklerkomponente Pyrimidin-Derivate mit 2 - 4 Aminosubstituenten und 0 - 2 Hydroxysubstituenten am Pyrimidin-Ring verwendet. Diese Pyrimidin-Derivate, wie beispielsweise 2,4,5,6-Tetraaminopyrimidin, 2-Hydroxy-4,5,6-triaminopyrimidin, 4-Hydroxy-2,5,6-triaminopyrimidin und 2,4-Dihydroxy-5,6-diaminopyrimidin, sind dem Fachmann bekannte Verbindungen. Stellvertretend für eine Vielzahl von Druckschriften, die diese Verbindungen offenbaren, wird auf die DE-OS 23 59 399 sowie die Monographie von Kh. Schrader, Grundlagen und Rezepturen der Kosmetika, Dr. Alfred Hüthig Verlag, Heidelberg, 2. Auflage, verwiesen.

Insbesondere auf den Inhalt der DE-OS 23 59 399, soweit er die Herstellung der Verbindungen offenbart, wird ausdrücklich Bezug genommen.

Besonders bevorzugte Pyrimidin-Derivate sind, sowohl im alleinigen Einsatz als auch als Kombination, 2,4,5,6-Tetraaminopyrimidin und 4-Hydroxy-2,5,6-triaminopyrimidin.

Bezüglich der Komponente 1-(2'-Hydroxyethyl)-2,5-diaminobenzol wird insbesondere auf die Offenbarung der DE-A1-39 17 304 verwiesen, die diese Komponente unter der Bezeichnung 2-(2,5-Diaminophenyl)ethanol beschreibt und auch deren Verwendung in Haarfärbemitteln offenbart.

Aus dieser Druckschrift können aber keine Hinweise auf die erfindungsgemäße Kombination entnommen werden.

Die in den erfindungsgemäßen Mitteln enthaltenen Entwickler- und Kupplerkomponenten können sowohl als freie Basen als auch in Form ihrer anorganischen oder organischen Salze, z.B. der Hydrochloride, der Sulfate und Hydrobromide, eingesetzt werden.

Weiterhin wurde gefunden, daß Färbungen mit überraschend guten Eigenschaften erzielt werden, wenn die erfindungsgemäßen Mittel als Kupplerkomponenten m-Aminophenole der Formel (I) enthalten sind, 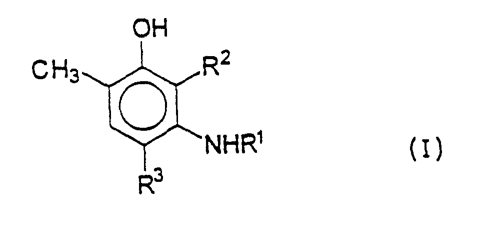 in der R¹ steht für Wasserstoff, eine Methylgruppe, eine Ethylgruppe oder eine Hydroxyalkylgruppe mit 2 oder 3 Kohlenstoffatomen und R² und R³ unabhängig voneinander stehen für Wasserstoff oder Chlor.

Besonders geeignete Kupplerkomponenten der Formel (I) sind 2-Methyl-5-amino-phenol, 2-Methyl-5-β-hydroxyethylamino-phenol und 3-Amino-2-chlor-6-methylphenol.

Mittel, die neben den Kupplerkomponenten der Formel (I) noch bestimmte weitere Kupplerkomponenten enthalten, zeichnen sich ebenfalls durch unerwartet gute Färbeergebnisse aus. Diese weiteren Kupplerkomponenten werden bevorzugt ausgewählt aus
- Resorcin und Resorcin-Derivaten,
- Naphthalin-Derivaten mit mindestens einer OH-Gruppe sowie
- Pyridin-Derivaten mit mindestens einer C₁₋₄-Alkylgruppe und mindestens einer Hydroxygruppe.

Ganz besonders geeignete weitere Kupplerkomponenten sind Resorcin, 2-Methylresorcin, 5-Methylresorcin, 2,5-Dimethylresorcin, 4-Chlorresorcin, 1,5-, 1,7- und 2,7-Dihydroxynaphthalin sowie 2,6-Dihydroxy-3,4-dimethylpyridin.

Die erfindungsgemäßen Haarfärbemittel enthalten sowohl die Entwicklerkomponenten als auch die Kupplerkomponenten bevorzugt in einer Menge von 0,01 bis 20 Gew.-%, vorzugsweise 0,5 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

Neben den erfindungsgemäßen Entwickler- bzw. Kuppler-/Entwickler-Kombinationen können die Haarfärbemittel gewünschtenfalls weitere Kupplerund/oder Entwicklerkomponenten enthalten, um spezielle Farbnuancen zu erhalten. Geeignete Verbindungen wurden bei der Diskussion des Standes der Technik bereits genannt.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe, z.B. aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Nitro-blau, Disperse Blue 3, Basic Blue 99, HC Violet 1, Disperse Violet 1, Disperse Violet 4, Disperse Black 9, Basic Brown 16, Pikraminsäure und Rodol 9 R, bekannten Verbindungen, in einer Menge von 0,01 bis 20 Gew.-%, bezogen auf das gesamte Oxidationshaarfärbemittel.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel werden bevorzugt auf einen pH-Wert von 7 bis 11, insbesondere von 9 bis 10 eingestellt.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonatoder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Diund Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO3H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -C00(-)- oder -SO₃(-)-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C8-C18-Alkyl- oder -Acylgruppe im Molekül mindestens eine freie Aminogruppe und mindestens eine -COOH- oder -S03H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin,
- C8-C22-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{R} vertriebenen Dialkylammoniummethosulfate und Methyl-hydroxyalkyl-dialkoyloxyalkyl-ammoniummethosulfate.

Ein Beispiel für ein als kationisches Tensid einsetzbares quaternäres Zuckerderivat stellt das Handelsprodukt Glucquat^{R}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Farbstoffe zum Einfärben der Zubereitungen,
- Antischuppenwirkstoffe wie Piroctone Olamine und Zink Omadine,
- Alkalisierungsmittel wie beispielsweise Ammoniak, Monoethanolamin, 2-Amino-2-methylpropanol und 2-Amino-2-methyl-propandiol-1,3.
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N20, Dimethylether, C02 und Luft,
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen insbesondere Wasserstoffperoxid oder dessen Anlagerungsprodukte an Harnstoff, Melamin oder Natriumborat. Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zu Verstärkung der Wirkung einer geringen Mengen vorhandener Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern.

### Beispiele

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol^{R}techn.¹ | 4,0 |
| Texapon^{R}N 28² | 40,0 |
| Dehyton^{R}K³ | 25,0 |
| Eumulgin^{R}B 2⁴ | 1,5 |
| destilliertes Wasser | 12,5 |

| | |
|---|---|
| ¹ C₁₂₋₁₈-Fettalkohol (HENKEL) | |
| ² Natriumlaurylethersulfat (ca. 28 % Aktivsubstanz; CTFA-Bezeichnung: Sodium Laureth Sulfate) (HENKEL) | |
| ³ Fettsäureamid-Derivat mit Betainstruktur der Formel R-CONH(CH₂)₃N⁺(CH₃)₂CH₂COO⁻ (ca. 30 % Aktivsubstanz; CTFA-Bezeichnung Cocoamidopropyl Betaine) (HENKEL) | |
| ⁴ Cetylstearylalkohol mit ca. 20 Mol EO (CTFA-Bezeichnung: Ceteareth-20) (HENKEL) | |

Auf Basis dieser Creme wurde dann folgende Haarfärbecremeemulsion hergestellt:

| | |
|---|---|
| Cremebasis | 50,0 |
| Entwicklerkomponenten | s. Tabelle 1 |
| Kupplerkomponenten | s. Tabelle 1 |
| Na₂SO₃ (Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit 3 %iger Wasserstoffperoxidlösung als Oxidationslösung durchgeführt. Hierzu wurden 100 g der Emulsion mit 50 g Wasserstoffperoxidlösung (3 %ig) versetzt und vermischt.

Zur Bestimmung der Färbeeigenschaften dienten sogenannte "Egalisiersträhnen", die wie folgt hergestellt wurden.

Die abgebundene untere Hälfte einer ca. 2 g schweren hellgrauen Kerling-Haarsträhne von ca. 16-18 cm Länge wurde mit 7 g eines handelsüblichen Wellmittels (Poly^{R} Lock Welgel) bei 27 °C in einem Umlufttrockenschrank 30 Minuten behandelt. Anschließend wurde dieselbe Strähnenhälfte mit warmem Wasser gespült und dann 10 Minuten mit 7 g einer handelsüblichen Fixierlösung (Poly^{R}Lock Fixieremulsion) behandelt, ausgewaschen und mittels eines Heizlüfters getrocknet. Danach wurde die so behandelte Strähnenhälfte mit 4g eines handelsüblichen Blondiermittels (Poly^{R} Blond Ultra, bestehend aus 1 Teil Poly Blond Blondiercreme, 1 Teil Poly Blond Blondierdispersion Ultra und 0,28 Teilen Blondaktivator) 30 Minuten bei 27 °C ultrablondiert. Die Strähnenhälfte wurde sodann erneut gewaschen, getrocknet und nochmals, wie oben beschrieben, einer Kaltwellbehandlung unterzogen. Zuletzt wurde dann die gesamte Haarsträhne mit 8 g des oben genannten Blondiermittels 30 Minuten bei 27 °C ultrablondiert. Die so behandelten Strähnen kamen frühestens nach einer Ruhepause von 2 Wochen zur Ausfärbung.

Zur Ausfärbung wurde dann die Färbecreme auf diese Egalisiersträhnen aufgetragen und dort 30 Minuten bei 27 °C belassen. Nach Beendigung des Färbeprozesses wurde das Haar gespült, mit einem üblichen Haarwaschmittel gewaschen und anschließend getrocknet. Lediglich solche Strähnen, die zur Beurteilung der Waschechtheit dienten, wurden nach dem Färbeprozeß vor dem Trocknen nicht mehr gewaschen. Der Nuancenausfall der Ausfärbungen wurde mittels Munsell- und Deutschem Farbatlas visuell bestimmt.

Die untersuchten Entwickler- und Kuppler-Kombinationen sowie die erhaltenen Färbeergebnisse im Rahmen der oben aufgeführten Rezepturen sind in Tabelle 1 zusammengestellt (Mengenangaben in g).

**Tabelle 1:**

| | 1 | 2 | 3 |
|---|---|---|---|
| Entwickler-Komponenten: | | | |
| - 1-(2'-Hydroxyethyl)-2,5-diamino-benzol-sulfat | 0,14 | 0,71 | 0,80 |
| - 2,4,5,6-Tetraaminopyrimidin-sulfat | 2,75 | 0,46 | 0,57 |
| - p-Aminophenol-hydrochlorid | - | 0,16 | 0,21 |
| | | | |

| Kuppler-Komponenten: | | | |
|---|---|---|---|
| - 2-Methyl-5-aminophenol | 0,04 | - | 0,02 |
| - 2,6-Dihydroxy-3,4-dimethylpyridin | 0,66 | - | - |
| - 2,7-Dihydroxynaphthalin | - | 0,11 | 0,12 |
| - 2-Methylresorcin | 0,48 | 0,35 | 0,06 |
| - 4-Chlorresorcin | - | 0,13 | 0,14 |
| - Resorcin | 0,21 | 0,12 | 0,11 |
| - 2-Aminomethyl-3-amino-6-methoxypyridin-dihydrochlorid | 0,07 | - | - |
| - m-Aminophenol | - | 0,05 | 0,05 |
| | | | |
| Nuance | granatrot | mahagony | kastanie |
| | | | |
| Waschbeständigkeit nach 6 Shamponierungen | 2 | 1 | 1-2 |
| | | | |
| Egalisierung nach 6 Shamponierungen | 2 | 1-2 | 1-2 |
| (Notenskala: 1 = sehr gut, 5 = sehr schlecht) | | | |

## Patentansprüche

1. Oxidationsfärbemittel zum Färben von Keratinfasern enthaltend Kupplerkomponenten und Entwicklerkomponenten in einem wasserhaltigen Träger, **dadurch gekennzeichnet, daß** als Entwicklerkomponente mindestens ein Pyrimidin-Derivat mit 2 - 4 Aminosubstituenten und 0 - 2 Hydroxysubstituenten am Pyrimidin-Ring in Kombination mit 1-(2'-Hydroxyethyl)-2,5-diaminobenzol enthalten ist.

2. Oxidationsfärbemittel nach Anspruch 1, **dadurch gekennzeichnet, daß** das Pyrimidin-Derivat ausgewählt ist aus 2,4,5,6-Tetraaminopyrimidin und 4-Hydroxy-2,5,6-triaminopyrimidin.

3. Oxidationsfärbemittel nach Anspruch 1 oder 2, **dadurch gekennzeichnet, daß** als Kupplerkomponenten m-Aminophenole der Formel (I) enthalten sind, 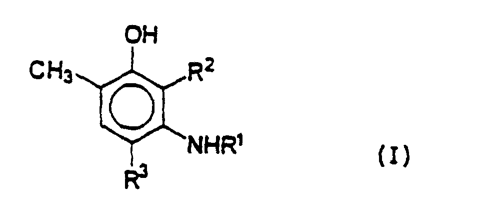 in der R¹ steht für Wasserstoff, eine Methylgruppe, eine Ethylgruppe oder eine Hydroxyalkylgruppe mit 2 oder 3 Kohlenstoffatomen und R² und R³ unabhängig voneinander stehen für Wasserstoff oder Chlor.

4. Oxidationsfärbemittel nach einem der Ansprüche 1 bis 3, **dadurch gekennzeichnet, daß** eine weitere Kupplerkomponente enthalten ist, die ausgewählt ist aus
- Resorcin und Resorcin-Derivaten,
- Naphthalin-Derivaten mit mindestens einer OH-Gruppe sowie
- Pyridin-Derivaten mit mindestens einer C₁₋₄-Alkylgruppe und mindestens einer Hydroxygruppe.

5. Oxidationsfärbemittel nach Anspruch 4, **dadurch gekennzeichnet, daß** die weitere Kupplerkomponente ausgewählt ist aus Resorcin, 2-Methylresorcin, 5-Methylresorcin, 2,5-Dimethylresorcin, 4-Chlorresorcin, 1,5-, 1,7- und 2,7-Dihydroxynaphthalin sowie 2,b-Dihydroxy-3,4-dimethylpyridin.

6. Oxidationsfärbemittel nach einem der Ansprüche 1 bis 5, **dadurch gekennzeichnet, daß** Entwicklerkomponenten in einer Menge von 0,01 bis 20 Gew.-%, vorzugsweise 0,5 bis 5 Gew.-%, und Kupplerkomponenten in einer Menge von 0,01 bis 20 Gew.-%, vorzugsweise 0,5 bis 5 Gew.-%, jeweils bezogen auf das gesamte Oxidationsfärbemittel, enthalten sind.

7. Oxidationsfärbemittel nach einem der Ansprüche 1 bis 6, **dadurch gekennzeichnet, daß** mindestens ein direktziehender Farbstoff enthalten ist.

## Claims

1. Oxidation colorants for coloring keratin fibers containing primary intermediates and secondary intermediates in a water-containing carrier, **characterized in that** at least one pyrimidine derivative containing 2-4 amino substituents and 0-2 hydroxy substituents on the pyrimidine ring in combination with 1-(2'-hydroxyethyl)-2,5-diaminobenzene is present as the primary intermediate.

2. Oxidation colorants as claimed in claim 1, **characterized in that** the pyrimidine derivative is selected from 2,4,5,6-tetraaminopyrimidine and 4-hydroxy-2,5,6-triaminopyrimidine.

3. Oxidation colorants as claimed in claim 1 or 2, **characterized in that** m-aminophenols corresponding to formula (I): 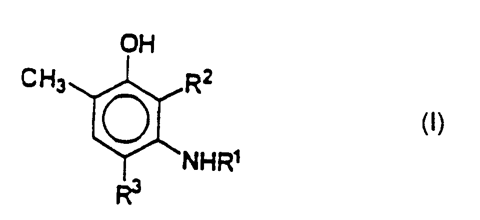 in which R¹ is hydrogen, a methyl group, an ethyl group or a hydroxyalkyl group containing 2 or 3 carbon atoms and R² and R³ independently of one another are hydrogen or chlorine,
are present as secondary intermediates.

4. Oxidation colorants as claimed in any of claims 1 to 3, **characterized in that** another secondary intermediate selected from
- resorcinol and resorcinol derivatives,
- naphthalene derivatives containing at least one OH group and
- pyridine derivatives containing at least one C₁₋₄ alkyl group and at least one hydroxy group
is present.

5. Oxidation colorants as claimed in claim 4, **characterized in that** the other secondary intermediate is selected from resorcinol, 2-methyl resorcinol, 5-methyl resorcinol, 2,5-dimethyl resorcinol, 4-chlororesorcinol, 1,5-, 1,7- and 2,7-dihydroxynaphthalene and 2,6-dihydroxy-3,4-dimethylpyridine.

6. Oxidation colorants as claimed in any of claims 1 to 5, **characterized in that** the primary intermediates are present in a quantity of 0.01 to 20% by weight and preferably in a quantity of 0.5 to 5% by weight while the secondary intermediates are present in a quantity of 0.01 to 20% by weight and preferably in a quantity of 0.5 to 5% by weight, based on the oxidation colorant as a whole.

7. Oxidation colorants as claimed in any of claims 1 to 6, **characterized in that** at least one substantive dye is present.

## Revendications

1. Colorant par oxydation en vue de teindre les fibres de kératine, contenant des composants de coupleur et des composants de révélateur dans un support contcnant de l'eau,
**caractérisé en ce que**
comme composant de révélateur, il contient au moins un dérivé de pyrimidine ayant de 2 à 4 substituants aminés et de 0 à 2 substituants hydroxy sur le cycle pyrimidine, en combinaison avec le 1-(2'-hydroxyéthyl)-2,5-diaminobenzène.

2. Colorant par oxydation selon la revendication 1,
**caractérisé en ce que**
le dérivé de la pyrimidine est choisi parmi la 2,4,5,6-tétraarninopyrimidine et la 4-hydroxy-2,5,6-triaminopyrimidine.

3. Colorant par oxydation selon la revendication 1 ou la revendication 2,
**caractérisé en ce qu'**
il contient comme composant de coupleur, des m-aminophénols de formule (I) 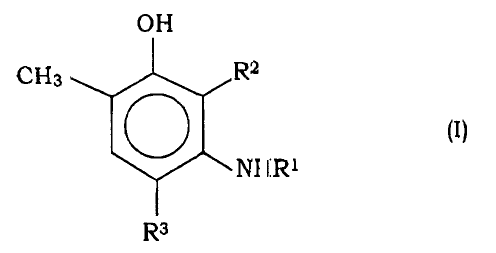 dans laquelle R¹ représente de l'hydrogène, un groupe méthyle, un groupe éthyle, ou un groupe hydroxy-alkyle ayant 2 ou 3 atomes de carbone et R² et R³, indépendamment l'un de l'autre, représentent de l'hydrogène ou du chlore.

4. Colorant par oxydation selon l'une quelconque des revendications 1 à 3,
**caractérisé en ce qu'**
il contient un autre composant de coupleur, qui est choisi parmi :
- le résorcinol et les dérivés du résorcinol,
- des dérivés du naphtalène ayant au moins un groupe OH ainsi que,
- des dérivés de pyridine ayant au moins un groupe alkyle en C₁-C₄ et au moins un groupe hydroxy.

5. Colorant par oxydation selon la revendication 4,
**caractérisé en ce que**
l'autre composant de coupleur est choisi parmi le résorcinol, le 2-méthylrésorcinol, le 5-méthylrésorcinol, le 2,5-diméthylrésorcinol, le 4-chlororésorcinol, les 1,5-, 1,7- et 2,7-dihydroxynaphtalènes ainsi que la 2,6-dihydroxy 3,4-diméthylpyridine.

6. Colorant par oxydation selon l'une des revendications 1 à 5,
**caractérisé en ce qu'**
il contient des composants de coupleur en une quantité allant de 0,1 à 20 % en poids, de préférence de 0,5 à 5 % en poids, et des composants de révélateur en une quantité allant de 0,01 à 20 % en poids, de préférence de 0,5 à 5 % en poids, à chaque fois rapporté à la totalité du colorant par oxydation.

7. Colorant par oxydation selon l'une des revendications 1 à 6,
**caractérisé en ce qu'**
il contient au moins un colorant montant directement.
